(19) Europäisches Patentamt
European Patent Office
Office européen des brevets

(11) **EP 2 433 602 B1**

(12) **EUROPEAN PATENT SPECIFICATION**

(45) Date of publication and mention
of the grant of the patent:
**17.08.2016 Bulletin 2016/33**

(51) Int Cl.:
*A61F 13/49* *(2006.01)*    *A61F 13/15* *(2006.01)*
*A61F 13/53* *(2006.01)*

(21) Application number: **10777689.0**

(22) Date of filing: **12.05.2010**

(86) International application number:
**PCT/JP2010/058044**

(87) International publication number:
**WO 2010/134456 (25.11.2010 Gazette 2010/47)**

(54) **ABSORBENT BODY AND ABSORBENT ARTICLE**

SAUGFÄHIGER KÖRPER UND SAUGFÄHIGER ARTIKEL

CORPS ABSORBANT ET ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **20.05.2009 JP 2009122491
27.05.2009 JP 2009128246
04.06.2009 JP 2009135570**

(43) Date of publication of application:
**28.03.2012 Bulletin 2012/13**

(73) Proprietor: **Kao Corporation
Chuo-Ku
Tokyo 103-8210 (JP)**

(72) Inventors:
• **KOUTA, Takuya
Haga-gun
Tochigi 321-3497 (JP)**
• **KAWAZOE, Masashi
Haga-gun
Tochigi 321-3497 (JP)**
• **HAYASHI, Yuka
Haga-gun
Tochigi 321-3497 (JP)**
• **NAGAHARA, Shinsuke
Haga-gun
Tochigi 321-3497 (JP)**

(74) Representative: **Vossius & Partner
Patentanwälte Rechtsanwälte mbB
Siebertstrasse 3
81675 München (DE)**

(56) References cited:
**EP-A1- 0 947 549      EP-A1- 1 621 166
EP-A1- 2 014 269      EP-A1- 2 226 046
JP-A- 3 502 531      JP-A- 10 121 301
JP-A- 10 314 217      JP-A- 11 216 161
JP-A- H05 505 533      JP-A- 2009 072 421
JP-A- 2009 136 498      JP-A- 2009 172 363
JP-A- 2010 124 900**

**Description**

TECHNICAL FIELD

The present invention relates to an absorbent body that is used for absorbent articles such as sanitary napkins, disposable diapers and incontinence pants; and an absorbent article using the same.

BACKGROUND ART

An absorbent article that is used for sanitary napkins, disposable diapers and the like generally has an absorbent body that absorbs and retains a liquid or the like between a liquid permeable topsheet that is positioned on the side of skin and a liquid impermeable backsheet that is positioned on the side of a garment. The absorbent body generally has an approximate rectangular form that covers excretion points of a wearer, and the absorbent body having improved shape and structure have been proposed.

For example, Japanese patent No. 3558801 discloses a flat type diaper comprising an absorbent body of a size that broadly covers the entirety of front and rear excretion areas from a gluteal region to a abdomen region, the entirety of one side surface of which is fixed on an outer sheet; and a plurality of notched thin portions that are disposed in parallel in the center of the absorbent body. In this diaper, a stringy elastic member is disposed orthogonally to the parallel direction of the notched thin portions of the above-mentioned absorbent body, whereby the notched thin portions are deformed so as to be opened and closed during wearing to provide some stretchability and bending property to the entirety of the absorbent body. However, this absorbent body is not composed of a plurality of independent absorbent units. Since the entirety of the large absorbent body is fixed on the sheet, the diaper is not so different from general ones in this point, and thus significant improvement of fittability to body and followability to motion against the motion of a body cannot be expected.

Japanese patent No. 2703596disclose an absorbent pad in which a liquid impermeable back sheet and a liquid permeable cover sheet are joined to each other at a rhombic tone lattice-shaped (Narihira lattice-shaped) line. The main body part of an absorbent material is disposed in an enclosed state in each of rhombic areas surrounded by the joining line. It is considered that the joining line forming an orthogonal lattice-shape on which the above-mentioned absorbent material is absent becomes a water conduit and a liquid may flow therein. However, the whole surface of the main body part of the above-mentioned absorbent material is fixed on the liquid impermeable back sheet, and thus it is also difficult to expect significant improvement of fittability to body and followability to motion. Furthermore, the lattice-shaped joining line that constitutes the above-mentioned water conduit may rather lead to a residual liquid or may be an interceptive element for liquid transmission between the lattice-shaped areas depending on the purpose of use and the state of excrement.

In an article described in Japanese patent No. 4173656, a plurality of rectangular tabular absorbent bodies are arrayed in parallel so that their longitudinal directions are oriented in the same direction on a stretchable sheet, and one longitudinal side of each absorbent body is fixed on the stretchable sheet by adhesion. The respective absorbent bodies are disposed so that they are overlapped with each other in the width direction, and are connected by a sufficient overlap amount so that the overlapping state of the absorbent bodies is maintained even when the stretchable sheet is stretched. Since each of the tabular absorbent bodies is fixed on the stretch sheet only at one longitudinal side, stretchability in the direction orthogonal to the longitudinal side is assured more than the case when the entirety of the absorbent bodies that covers the whole area of the stretch sheet is fixed on the whole surface. However, stretchability in the longitudinal side direction is not improved and not different from those of conventional ones. The same is true on bending property, and complex deformation cannot be followed.

JP-A-2003-103677 discloses an absorbent body product that is constituted by a composite sheet composed of a laminate of a nonwoven fabric layer and a fiber web layer as a topsheet; and an absorbent body. An example of the composite sheet is composed of: a first network area as lattice-shaped belt-like areas in a planar view, which has a high density and a low thickness; and a second network area as a rectangular area separated by the above-mentioned lattice-shaped areas, which has a low density and a high thickness. The above-mentioned first network area is formed as an area having a higher density than that of the second network area by compression-bonding a bulky fiber web with a nonwoven fabric layer on the area. Namely, nonwoven fabric fibers are in a crushed state flatly in the first network area. However, the fiber web is disposed on the nonwoven fabric layer on the whole surface in this composite sheet, and thus the basis weight is not varied as a whole, and the composite sheet is not divided. Therefore, improvements of stretchability, and fittability and followability to a body are highly limited.

Further related technology is shown in EP 2 014 269 A1, or EP 0 947 549 A1.

## SUMMARY OF INVENTION

### TECHNICAL PROBLEM

In view of the problems of the above-mentioned conventional arts, the present inventors have developed an absorbent body that has extremely high "fittability to human body", i.e., the entirety of the absorbent body deforms flexibly and fits the complexly undulating surface of the skin of a wearer without any space, and also that has extremely fine "followability to motion", i.e., the absorbent body finely follows the deformation due to the motion of the wearer (see Japanese Patent Application No. 2007-316239 [JP-A-2009-136498], and Japanese Patent Application No. 2007-332419 or its counterpart EP 2 226 046 A1 (prior art under Art. 54(3) EPC only)). The object of the present invention is to provide: an absorbent body in which retention of absorption of a liquid or the like during deformation of the thus-newly is improved;, and an absorbent article therewith.

### SOLUTION TO PROBLEM

The invention is specified in the claims.

### ADVANTAGEOUS EFFECTS OF INVENTION

The absorbent body of the present invention has extremely high "fittability to human body" and "fllowability to motion", and also realizes high retention of absorption for a liquid or the like even when it is deformed by deformations such as stretch and flexion.

### BRIEF DESCRIPTION OF DRAWINGS

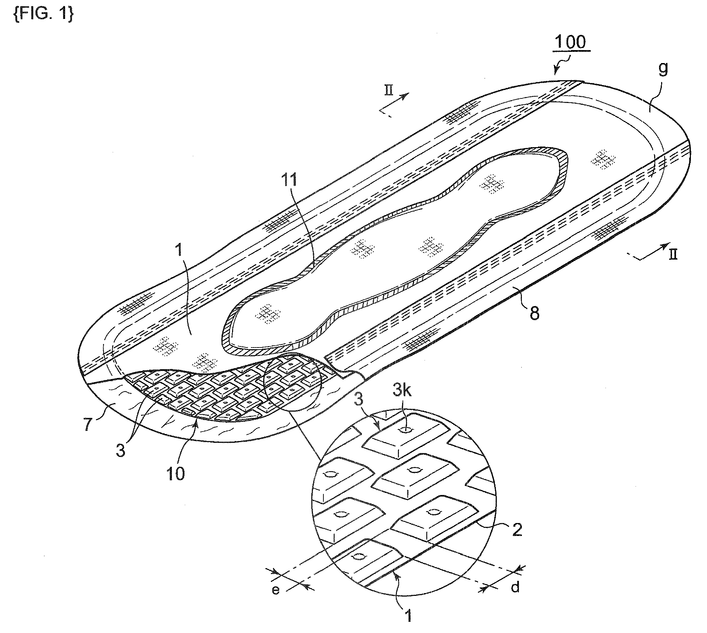

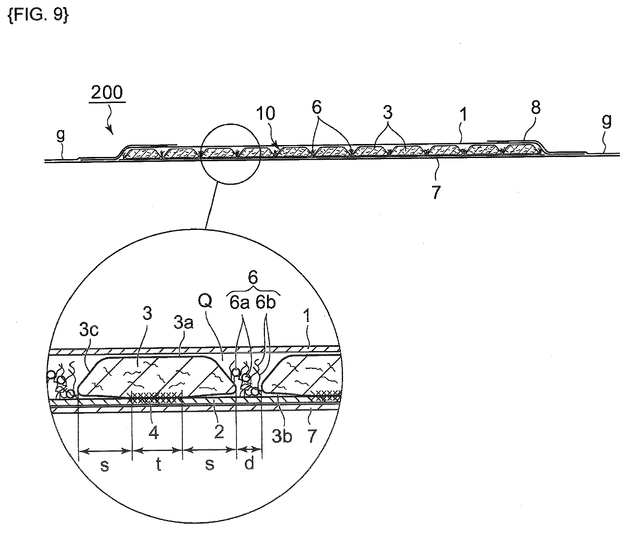

{FIG. 1}
FIG. 1 is a partially cutaway perspective view that schematically shows the absorbent article (sanitary napkin) using the absorbent body according to an embodiment (the first embodiment) of the present invention.
{FIG. 2}
FIG. 2 is a cross-sectional view along the line II-II of FIG. 1.
{FIG. 3}
FIG. 3 is a partial plane view that schematically shows the absorbent body according to an embodiment of the present invention.
{FIG. 4}
FIG. 4 is a partial cross-sectional view that shows the state during deformation as a model at the cross-sectional surface along the arrow view line IV-IV of FIG. 3 (b).
{FIG. 5}
FIG. 5 is a partial plane view that shows the absorbent body according to a modified example of the present embodiment in an area similar to that of FIG. 3.
{FIG. 6}
FIG. 6 is an enlarged plane view that shows the relationship between the sizes of the respective sites of the absorbent unit shown in FIG. 5.
{FIG. 7}
FIG. 7 is a plane view that shows (a) the natural length state and (b) the expanded state of the absorbent body test body prepared in Examples.
{FIG. 8}
FIG. 8 is a partially cutaway perspective view of the absorbent article (sanitary napkin) using the absorbent body according to an embodiment (the second embodiment) of the present invention.
{FIG. 9}
FIG. 9 is a cross-sectional view along the line IX-IX of FIG. 1.
{FIG. 10}
FIG. 10 is a plane view that shows the natural length state and stretched state of the absorbent body test body prepared in Examples.
{FiG. 11}
FIG. 11 is a cross-sectional view that shows, as a model, the behavior of the absorbent article according to an embodiment of the present invention when a pressure is applied to the topsheet thereof.

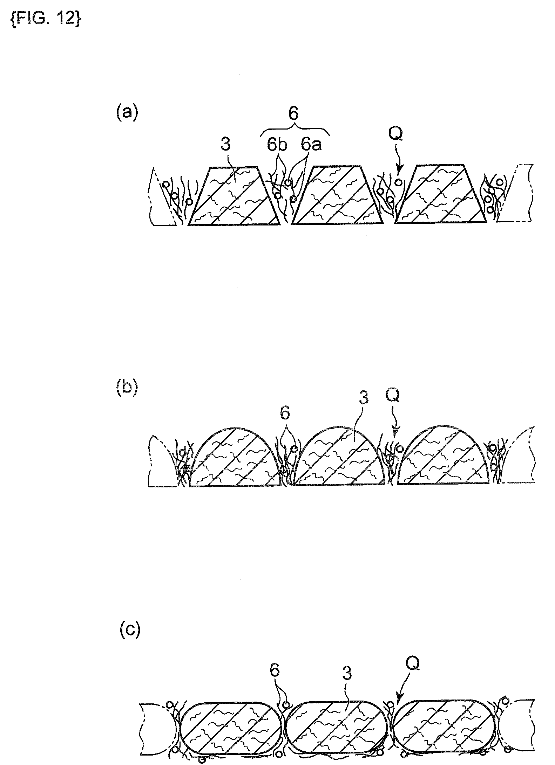

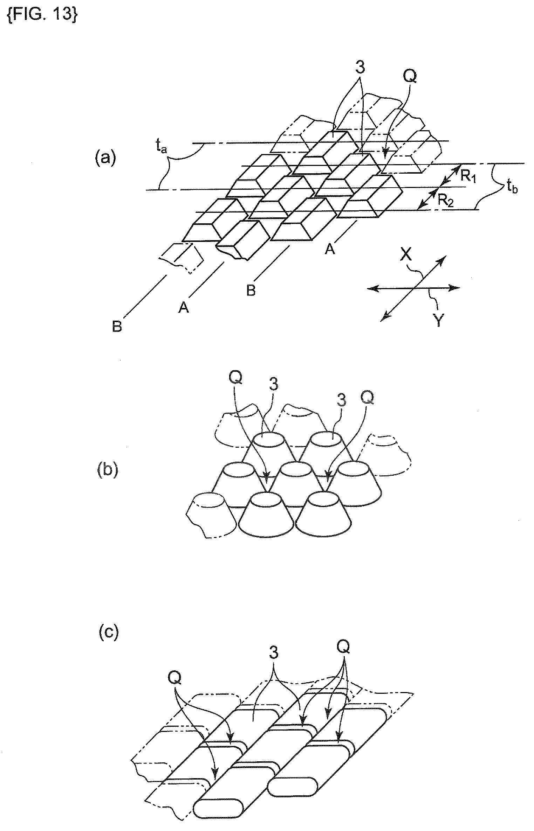

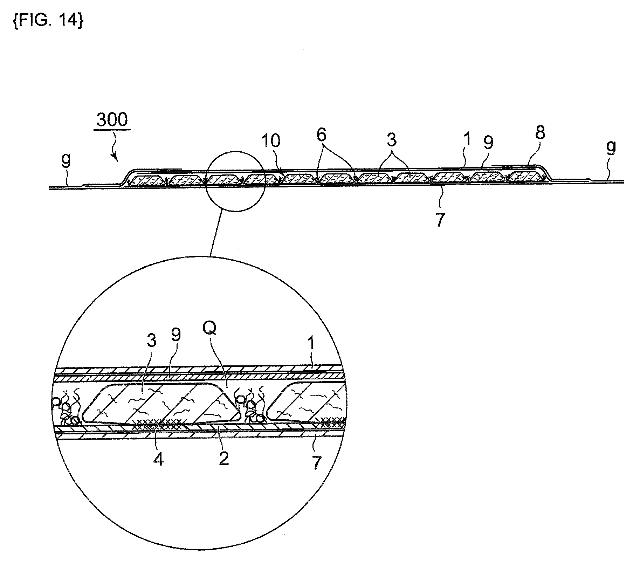

{FIG. 12}
FIG. 12 is an enlarged cross-sectional view that schematically shows the states of the absorbent units and absorbent material of the absorbent article according to an embodiment of the present invention.
{FIG. 13}
FIG. 13 is an enlarged perspective view that schematically shows the arrangement of the absorbent units of the absorbent article according to an embodiment of the present invention.
{FIG. 14}
FIG. 14 is a cross-sectional view that schematically shows a sanitary napkin according to a further different embodiment (the third embodiment) of the absorbent article of the present invention.

DESCRIPTION OF EMBODIMENTS

Hereinafter, the present invention is described in detail with reference to the drawings based on preferred embodiments thereof.

(First embodiment)

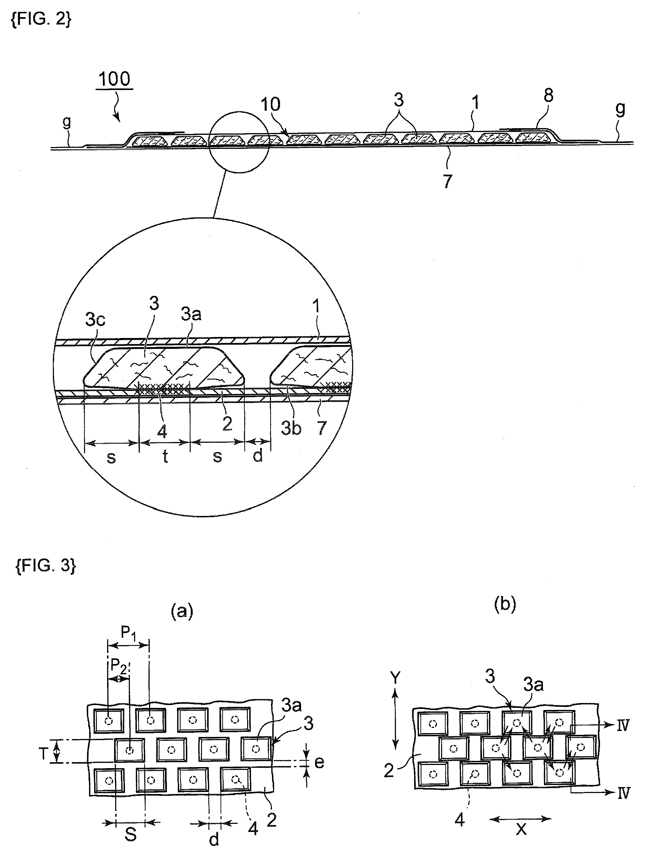

FIG. 1 is a partially cutaway perspective view of an absorbent article (sanitary napkin) 100 using the absorbent body according to the first embodiment of the present invention, and FIG. 2 is a cross-sectional view along the line II-II of FIG. 1. The absorbent body 10 of the present embodiment comprises a stretchable base sheet 2 and many independent absorbent units 3 that are disposed on the surface (upper surface) of the seat 2 in the longitudinal and transverse directions so that they have predetermined gaps d and e with each other in a natural state. The absorbent unit 3 is rectangular in a planar view, and is a landscape trapezoid and a slightly-rounded truncated square pyramid (truncated pyramid) shape in the cross-sectional surface. However, the shape is not specifically limited in this embodiment, and any shape can be adopted. In the present embodiment, the absorbent units 3 are comprised of pulp fibers and superabsorbent polymers, and the outlines thereof are not necessarily standard forms as illustrated, and are preferably of the steric shape as mentioned above as a whole.
The absorbent unit 3 has a trapezoidal shape in the cross-section, and the area of the top surface 3a is slightly smaller than the area of the bottom surface 3b. Furthermore, in the cross-sectional surface the side 3c thereof are inclined (sloped) toward the bottom surface so as to spread wide toward the end. In each absorbent unit 3, a part of the bottom surface thereof rather than the whole of the bottom surface, specifically only a fixing unit 4 that is positioned in the area of the absorbent unit in a planar view (see the center part t of the bottom surface 3b in the enlarged view of FIG. 2) is fixed on the base sheet 2. In other words, in case where the projection images of the absorbent unit 3 and fixing unit 4 are projected in the thickness direction of the absorbent body, there is a relationship in which the projection image of the fixing unit 4 is enclosed in the projection image of the absorbent unit 3. The fixing unit 4 is designed to not exhibit stretchability. The fixing unit 4 can be formed by, for example, ultrasonic embossing, whereby the stretchability of the base sheet 2 is lost on this part. Alternatively, the fixing unit 4 may be formed by a hot-melt adhesive material or the like. At this case, in order to make the attachment between the absorbent unit 3 and base sheet 2 on the fixing unit stronger and more consolidated, a pressure may be applied by using a pin from the skin surface 3a of the absorbent unit 3. By applying a pressure by using a pin, a dimpled portion 3k is formed on the center of the absorbent unit of the present embodiment (see FIG. 1). Since the dimpled portion 3k gets a high density, a sparse-dense gradient is formed between the dimpled portion 3k and other part, whereby a liquid is readily drawn in the center, and thus absorbability is improved. In addition, the bottom surface 3b of the absorbent unit 3 is in the state that it is not adhered to the base sheet at the outer of the fixing unit 4, whereby the stretchability of the base sheet 2 is maintained in the outer side area s that corresponds to the bottom surface 3b.
As mentioned above, since the part attached to the base sheet 2 is only the area t that corresponds to only the fixing unit 4, the shape and function of each absorbent unit 3 is hardly affected by the stretching of the base sheet and stable absorbing performance can be exerted. On the other hand, from the viewpoint of the function of the base sheet, since the respective absorbent units 3 are present independently as mentioned above, the motion of the base sheet is not constrained excessively, and the entirety of the base sheet 2 can stretch and bend extremely flexibly. As a result, the deformability of the entirety of the absorbent body against the motion of a wearer is ensured, and furthermore, according to the present embodiment, the absorbent units 3 allow liquid current to pass with each other during stretching and bending, whereby highly stable absorbability can be maintained during wearing as mentioned below.
Specifically, for example, when the sanitary napkin 100 shown in FIG. 1 is expanded in the longitudinal direction, the absorbent units 3, 3, 3 ... are further separated from each other in the longitudinal direction, i.e., the interval d is increased. On the other hand, the width direction of the sanitary napkin 100 approaches as if it were shrank and the interval e becomes narrow. By this manner, the absorbent units 3, 3, 3 ... maintain coordination for transmission of a liquid or the like each other while the entirety of the absorbent body 10 is deformed, whereby show fine retention of absorbability of the liquid or the like. In the present invention, the liquid-current-to-pass means that, when an elongation force is applied to the absorbent body, the absorbent units approach or contact with each other and are in the state that a liquid or the like can be transferred between the absorbent units. Although the distances (gaps d and e) between the absorbent units in the state that such elongation force is applied are not specifically limited, the distances preferably approach to from 0 to 30 mm, more preferably approach to from 0 to 10 mm by a general elongation force (for example, 100 cN/25 mm) that is caused during wearing. Furthermore, it is further preferable that the distances approach to from 0 to 5 mm.

Specifically, since the absorbent body 10 of the present embodiment is constituted by an assembly of many small independent absorbent units, the "fittability to body for fitting undulation (unevenness) of a skin surface is increased significantly as compared to that of conventional ones. Furthermore, since the absorbent body finely follows the motion of a wearer, it has extremely fine "motion followability to motion" by which generation of partial gaps against the skin, and the like are prevented. According to the present embodiment, such fine deformability and fine retainment of absorption of a liquid or the like that is not affected by the deformation can be achieved simultaneously.

Furthermore, in the present embodiment, since many small absorbent units that are comparted as mentioned above are disposed on the base sheet by a specific arrangement, the absorbent units also preferably fit a skin surface that bends extremely complexly. For example, when a sanitary napkin is applied to from an excretion point to a gluteal region, it curves in the longitudinal direction along the roundness of buttocks. However a conventional absorbent body that is enclosed therein generally has significantly higher stiffness than that of other elements and thus is sometimes buckled. Due to the relationship with this buckling deformation, the inside absorbent body is difficult to get into a gluteal cleft, and a large gap is sometimes formed therein. Namely, buckling in the longitudinal direction and flexion in the width direction are difficult to be achieved simultaneously, and thus sufficient fitting property is difficult to be obtained. Therefore, menstrual blood or the like is readily transferred via the space formed in the gluteal cleft, which causes leakage toward the back direction during sleeping.

On the other hand, the sanitary napkin to which the absorbent body of the present embodiment is applied gently curves and fits the roundness of buttocks, and also suitably gets into and fit a gluteal cleft, i.e., deforms into a so-called saddle surface shape or hyperbolic paraboloid shape to fit the gluteal cleft while providing little gap. Therefore, extremely fine fittability, i.e., the napkin is in abutting contact with the skin surface of a wearer from the vicinity of an exception point to a gluteal region almost without any slight gap, is realized. Furthermore, since the napkin follows the motion of the skin surface when a wearer rolls over or walks, a gap is not formed and fine fitting property is maintained.

Furthermore, since the sanitary napkin of the present embodiment has extremely high shape-restoring property, for example, when the napkin is folded in three or the like in an individual packaging, folding lines formed by doing so are hard to remain, and thus the above-mentioned fine fittability to body and followability can be obtained from initiation of wearing.

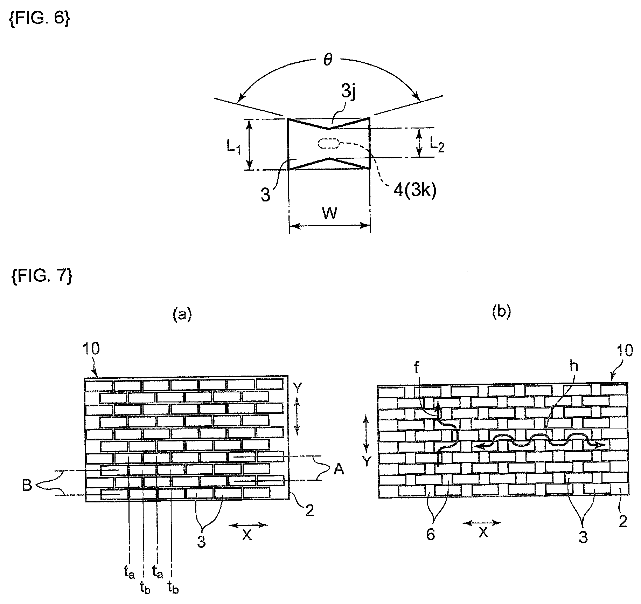

In the absorbent body 10 of the present embodiment, the absorbent units 3 are disposed by a staggered arrangement. In the present embodiment, the staggered arrangement means an arrangement in which rows of a plurality of absorbent units are disposed parallel and the pitches of the absorbent units on the adjacent rows are shifted each other. In other word, the arrangement is a disposition in which when the absorbent units 3 of a certain row are projected in the orthogonal direction, they do not correspond to the projection images of the absorbent units 3 adjacent to the above row. When a specific example thereof is explained with referring to FIG. 7, an absorbent unit row A is adjacent in parallel to an absorbent unit row B that extends in the longitudinal direction (direction X), and the rows are repeated alternately. Auxiliary lines $t_b$ that are continued from the center of the absorbent unit rows B in a planar view in the direction orthogonal to the direction to which the absorbent unit rows B are extended (direction Y) are assumed. On the other hand, similar auxiliary lines $t_a$ in the absorbent unit rows A are assumed. The auxiliary lines $t_a$ and auxiliary lines $t_b$ are arranged so that they are shifted by half pitches with each other, i.e., so that the intervals between the auxiliary lines $t_a$ and the auxiliary lines $t_b$ are equal as a whole, to constitute a staggered arrangement. Examples of a modified example of the present embodiment may include, for example, an embodiment in which the absorbent units rows A and absorbent units rows B are set in the width direction (direction Y) so as to provide displacement to auxiliary lines extending in the longitudinal direction (direction X), in which the direction to which the pitches are shifted is rotated by 90°. Furthermore, if the staggered arrangement is compared to stone masonry, it can be said to be coursed masonry, and examples of the modified examples thereof may include twill masonry, herringbone mansory, hexagonal masonry and the like.

In the sanitary napkin 100 of the present embodiment (see FIGS. 1 and 2), as mentioned above, the absorbent body 10 comprising the base sheet 2 and a plurality of the absorbent units 3 that are disposed thereon is disposed on a backsheet 7, and they are attached together by, for example, a hot-melt adhesive that is spirally applied. Furthermore, side sheets 8 are fixed on the backsheet 7 at the both side parts in the longitudinal direction of the backsheet by hot-melting or heat-sealing, and in the inner sides thereof topsheet 1 is held therebetween and fixed. In this case, the entirety of a peripheral edge g of the napkin is fixed by heat-sealing or the like so that the enclosed components of the absorbent body, a liquid that has been absorbed once, and the like are not leaked. The topsheet 1 is disposed on the absorbent body 10 so as to cover almost the whole surface of the absorbent body 10, and a groove 11 having a cloud-formed pattern in a planar view is formed on from the topsheet 1 to the absorbent body 10 by embossing to suppress the misorientation of the topsheet 1 and absorbent body 10 (the groove 11 is not illustrated in FIG. 2). The side of the topsheet 1 is to be contact with skin, and the side of the backsheet 7 is to be contact with an underwear.

FIG. 3 is a schematic partial plane view of the absorbent body according to the first embodiment of the present invention, in which (a) in the same drawing shows the state of the absorbent body 10 before deformation, and (b) in the same drawing shows the state when the absorbent body is extended in the longitudinal direction to cause deformation. Although the base sheet of the absorbent units may be wrinkled or twisted, it is not described in FIG. 3 (b). Furthermore, in the same drawing, dimples 3k are not shown and fixing units 4 are shown by broken lines.

FIG. 3 shows an arrangement of the absorbent units 3 in which the absorbent units 3 that are adjacent to each other in the transverse direction (direction Y) are disposed with half pitches $P_2$ shifted to the pitches $P_1$ in the longitudinal direction (direction X). When an absorbent article (not shown) comprising such an absorbent body 10 is worn, the base sheet 2 is sometimes deformed by twisting or sagging by a tensile strength applied in the longitudinal direction to the absorbent body 10. Due to this deformation by twisting, the gaps between the absorbent units 3 that are adjacent to each other in the transverse direction (direction Y) are decreased, and the absorbent units 3 in the transverse direction get close to each other, and the liquid on the absorbent body 10 may be transferred through the adjacent sites between the absorbent units 3 ranging transverse direction, as shown by the arrows in FIG. 3 (b). Therefore, the liquid is in the state that it may be transferred on the adjacent sites on between the absorbent units 3, whereby a network of the liquid-communicated parts is formed.

In the present embodiment, since each of the absorbent units 3 is fixed on the base sheet 2 only by the central fixing units 4 in a planar view as mentioned above, the planar shape of the entirety of the absorbent units 3 is hard to be affected even by twisting of or sagging in the absorbent body 10. Furthermore, the liquid absorbability of the absorbent body 10 is hard to be decreased, and stable absorbing property and liquid-transferring property are ensured during wearing by the above-mentioned liquid-current-to-pass. The direction and site for incorporating in the absorbent article may also be determined suitably in consideration of the direction and the like of occurrence of the liquid-current-to-pass during such stretch deformation. The size of the absorbent unit 3 in the embodiment 1 of FIG. 1 is exemplified to have a transverse width T of preferably from 1 to 50 mm, more preferably from 2 to 30 mm in view of utilization in sanitary napkins, but the size slightly differs according to the absorbent article to be incorporated. The longitudinal width S is preferably from 3 to 100 mm, more preferably from 5 to 80 mm. Furthermore, the above-mentioned pitch $P_2$ is preferably from 2 to 60 mm. The gaps d and e are preferably from 0.1 to 30 mm, and from 0.1 to 30 mm, respectively.

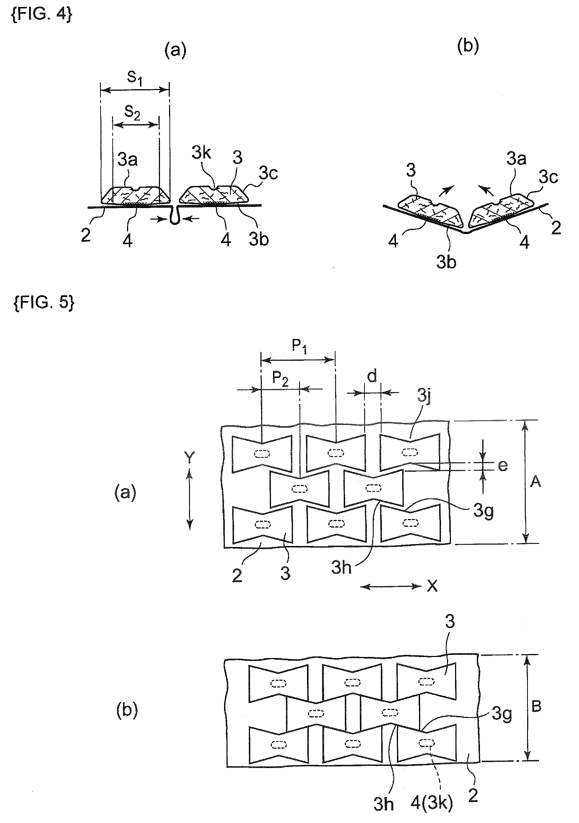

Since the side parts of each absorbent unit 3 in the present embodiment are inclined planes 3c, the absorbent units show fine fittability to a body, causes no uncomfortable feeling and fits to the body shape of an attached site even in a state in which the absorbent units 3 get close to each other. For example, in a model of an simplified shape of the cross-section, if a deformation force acts on the absorbent body 10, the absorbent units do not buffer each other and thus do not hinder fittability to body or followability to motion since the shoulders of the two absorbent units have been chamfered, even in the case that the absorbent units get close each other due to bending of the base sheet cured between the absorbent units as shown in FIG. 4 (a), as well as in the case that the absorbent units 3 are folded and get close each other as shown in FIG. 4 (b).

FIG. 5 is a schematic partial plane view that shows the absorbent body 10 of a modified example of the first embodiment of the present invention, and FIG. 6 is an enlarged view in which the absorbent unit is picked up and the relationship between the sizes is shown. In this embodiment, each absorbent unit 3 has a shape having a constriction in the middle in a planar view, and is specifically formed into a polygon (inverted hexagon) in which V-shaped bent parts 3j are disposed on the both sides (longitudinal sides) of a rectangle. Meanwhile, when a diagram in a planar view is defined in the present embodiment, the diagram also includes shapes that are obtained by chamfering the corners of the diagram, or rounded shapes. Furthermore, the present embodiment 2 has a staggered arrangement in which the constricted sides of the absorbent units 3 engage each other. Specifically, it has a staggered arrangement in which the oblique lines that are constricted in V-shapes of the inverted-hexagonal absorbent units are opposed to each other in parallel.

FIG. 5 (a) shows the state of the absorbent body before deformation, and FIG. 5 (b) shows the state in which the absorbent body is deformed by applying a tensile force. The respective absorbent units 3 on the base sheet 2 are paved in the longitudinal direction (direction X) and transverse direction (direction Y) on the base sheet 2, and are arranged in a staggered arrangement in which the absorbent units that are adjacent to each other in the transverse direction are arranged alternately in the transverse direction at intervals in the longitudinal direction of pitches $P_2$ that are approximately half of the pitches $P_1$ of the absorbent units in the longitudinal direction. In the natural state (relaxed state) in which the absorbent body 10 is not deformed by twisting or the like as shown in FIG. 5 (a), the respective absorbent units 3 have gaps d and e therebetween.

By disposing in such manner, when a tensile force in the longitudinal direction is applied to the absorbent body 10 in the state during wearing of the absorbent article comprising the absorbent body 10 of the present embodiment 2 to cause twisting or shrinking in the transverse direction (direction Y), and the transverse width A in the natural state becomes the transverse width B in the twisted or shrank state, the absorbent units 3 that are adjacent to each other in the transverse direction approach to each other at inclined planes 3g and 3h on one side of the V-shaped bent parts and are liquid-communicated with each other in a planar view. In this case, since the approaching or contacting area of the V-shaped inclined planes becomes longer as compared to those in the above-mentioned embodiment 1 and liquid-communicated is made at this long approaching area, finer transmission of a liquid or the like can be obtained. Furthermore, by disposing the absorbent units 3 each having such shape on the base sheet 2 in a staggered arrangement as illustrated, the absorbent body 10 becomes easy to be deformed in the longitudinal and transverse directions in a balanced fashion, whereby body fittability to body and followability to motion without unevenness and retention of absorption of a liquid or the like can be realized simultaneously.

The V-shaped bent part 3j on each of the opposed two side parts of the absorbent unit 3 in the present embodiment has a constriction angle $\theta$ of 90° or more and less than 180°, preferably of from 100° to 170°. Furthermore, in the present embodiment, the absorbent unit is formed so that $L_1 > L_2$ when the length between the volley bottoms of the constrictions is defined as $L_2$ and the length of side part that is orthogonal to the longitudinal direction (the direction X in the drawing) is defined as $L_1$. In addition, this polygonal absorbent unit 3 having V-shaped bent parts 3j is fixed on the base sheet 2 at only the fixing unit 4 on the center of the absorbent unit, whereas the absorbent unit is merely contacted with and is not fixed on the base sheet 2 on the rear surface outside of the fixing unit 4. In addition, the size of the absorbent unit in the present embodiment 2 has, for example, a transverse width $L_1$ of preferably 1 to 50 mm and $L_2$ of preferably from 1 to 50 mm in view of use as a sanitary napkin. The longitudinal width W is preferably about 3 to 100 mm.

As another modified example of the absorbent body of the present embodiment, it is possible that an absorbent material may be disposed on the space part between the absorbent unit 3 and absorbent unit 3 so that it has a lower basis weight than that of the absorbent units 3. In this case, the absorbent material preferably has components that are similar to the pulp fiber, absorbent polymer and the like that constitute the absorbent units 3, but the components are not necessarily limited to these.

The material for constituting the absorbent units 3 is not specifically limited, and fiber materials, porous materials, and combinations thereof and the like can be used. Examples of the fiber material that may be used include: natural fibers such as wood pulp, cotton and hemp; single fibers composed of synthetic resins including e.g. polyolefin-based resins such as polyethylene and polypropylene, polyester-based resins such as polyethylene terephthalate, and polyvinyl alcohol resins; conjugate fibers comprising two or more kinds of these resins; and semisynthetic fibers such as acetate and rayon. In the cases when fibers composed of a synthetic resin are used, the fibers may be heat-shrinkable fibers which deform by heat. For example, it is possible to employ fibers whose fineness is increased but whose fiber length is shorten by heat, or fibers whose fineness is changed little by heat but whose apparent length occupied by the fibers is shorten by deforming thereof into a coil shape. Examples of the porous materials that may be used include, sponges, nonwoven fabrics, and aggregates of high absorption polymers (i.e., aggregates of high absorption polymers and fibers), and the like can be used.

As the high-absorption polymer contained in the absorbent units 3, preferably include those that can absorb and retain a body fluid having a weight of five times or more of the own weight of the polymer, and can gel. The shape is not specifically considered, and may be a spherical, massive, botryoidal, powdery or fibrous form. The polymer is preferably in the form of particles having a size of preferably from 1 to 1,000 $\mu$m, more preferably from 10 to 500 $\mu$m. Examples of such high absorption polymer may include starch, crosslinked carboxylmethylated cellulose, polymers or copolymers of acrylic acid or alkali metal salts of acrylic acid, and the like, polyacrylic acid and salts thereof, and graft polymers of salts of polyacrylic acid. As the salt of polyacrylic acid, sodium salts can preferably be used. It is also possible to preferably use copolymers obtained by copolymerizing acrylic acid with a comonomer such as maleic acid, itaconic acid, acrylamide, 2-acrylamide-2-methylpropanesulfonic acid, 2-(meth)acryloylethanesulfonic acid, 2-hydroxyethyl(meth)acrylate or styrene sulfonic acid in a range in which the performance of the high-absorption polymer is not deteriorated.

In the case where the absorbent units 3 comprise a high absorption polymer, the ratio of the high absorption polymer in the weight of the absorbent units 3 is preferably from 5 to 95% by weight. In the case where the absorbent body 10 is used as an absorbent body for sanitary napkins or articles that are used for absorbing a liquid of a low excretion amount such as light incontinence, the ratio of the high absorption polymer in the weight of the absorbent units 3 is preferably from 10 to 30% by weight. In the case where the absorbent body 10 is used as an absorbent body for articles used for absorbing a liquid of a high excretion amount such as disposable diapers, the ratio of the high absorption polymer in the weight of the absorbent units 3 is preferably from 50 to 80% by weight.

Regardless of whether the absorbent unit 3 comprises the high absorption polymer or not, the stretchable absorbent body 10 preferably has a retention amount of 0.9%-by-wight aqueous sodium chloride solution of 0.1 g/g or more, specifically of 1 g/g or more since stable absorbing performance is exhibited even the base sheet 2 is stretched. In order to realize such retention amount, it is advantageous to use a combination of fibers having high hydrophilicity and high capillary force (for example, pulp, rayon and the like), synthetic fibers that do not wear out under moistness (that are not plasticized, or the moist strength thereof is not decreased) and the high absorption polymer as constitution materials for the absorbent units 3.

As the base sheet 2 on which the absorbent units 3 are fixed, one having functions of pulling in and diffusing the excretory liquid is used. Examples of the sheet having such functions may include; nonwoven fabrics, films, or porous bodies, and the like, which comprise fibers having hydrophilicity or comprise fibers that have been treated with a hydrophilic oil agent. These sheets may be in the state of a single layer, or may be of a multilayer structure comprising a plurality of layers that are laminated to form a single sheet. Of these, a sheet having stretchability is preferable, and the materials therefor can be used without specific limitations. Examples of such sheet may include nonwoven fabrics comprising fibers comprising an elastic resin as constitutional fibers (elastic nonwoven fabric), films comprising an elastic resin (elastic films), elastic porous bodies that are composed of an elastic resin in which a three-dimensional network has been formed in the structure by a means such as foaming, and the like. As the elastic nonwoven fabrics, elastic films and elastic porous bodies, those known in the art can be used. The base sheet 2 preferably has a basis weight of from 5 to 50 $g/m^2$, specifically of from 10 to 30 $g/m^2$.

It is preferable that the degree of stretchability of the base sheet 2 is 60% or above, and more preferably 80% or above, in stretch ratio which is measured as follows, from the standpoint of providing particularly favorable adaptability to a wearer's body and conformability to a wearer's movement. The stretch ratio is measured as follows. Measurement is carried out using a tension/compression tester RTC-1210A (supplied by Orientec Co., Ltd.) in the "tension mode". First, a measurement piece is sampled by cutting the base sheet 20 into a strip 25 mm wide and 150 mm long. The measurement piece is set between air chucks that are installed in the tension/compression tester at an initial sample length (chuck-to-chuck distance) of 100 mm, and the piece is extended by raising the chuck mounted to the load cell (rated output of 5 kg) of the tension/compression tester at a speed of 300 mm/min. When the measurement piece has been extended by a length 50% of the initial sample length, i.e., by 50 mm, the movement direction of the chuck is reversed, and the chuck is lowered at a speed of 300 mm/min and returned to the position of the initial sample length. During this operation, the relationship between the load detected by the load cell and the extension of the measurement piece is recorded in a chart, and the stretch ratio is obtained from the following equation (1) based on the chart.

$$\text{Stretch ratio} = \text{Recovery extension}/\text{Maximum extension length } (=50 \text{ mm}) \quad (1)$$

The "recovery extension" is defined as the distance the chuck has moved from the maximum extension length (=50 mm) at the time the load first becomes zero after starting to lower the chuck from the maximum extension length. Note that in cases where the measurement piece cannot extend up to the above-described size, measurement is carried out according to the following method.

<Measurement Piece>

Assuming that the length of the sheet in the chuck-to-chuck direction is L mm, the length of a section that is held is S mm, and the width of the sheet is C mm, a measurement piece is sampled by cutting the base sheet into a specimen (L+2S) mm × C mm wide in such a manner that the length ratio L:C becomes 3:5.

<Test>

The specimen is set to the tension/compression tester at a chuck-to-chuck distance of L, and the chuck is raised at a speed of 100.times.(L/30) mm/min until the measurement piece is extended by a length 50% of the initial sample length. The movement direction of the chuck is then reversed, and the chuck is lowered at a speed of 100.times.(L/30) mm/min and returned to the position of the initial sample length. Calculation is made according to the following equation (2):

$$\text{Stretch ratio} = \text{Recovery extension}/\text{Maximum extension length } (=L/2 \text{ mm}) \quad (2)$$

The topsheet is preferably composed of liquid permeable materials that provides soft feeling to skin. For example, it is possible to use nonwoven fabrics comprising natural fibers such as cotton as a material, and nonwoven fabrics comprising various synthetic fibers subjected hydrophilic treatment as a material. The backsheet is preferably composed of a liquid impermeable sheet material. The backsheet may be composed of a vapor permeable one as needed. In order to specifically obtain sufficient vapor permeability, it is preferable to use a porous film obtained by extending a film made of a synthetic resin such as polyethylene in which a micropowder composed of a filler such as calcium carbonate has been dispersed to form fine pores therein. Examples of the side sheet may include nonwoven fabrics, film sheets, paper and the like. In view of prevention of leakage, it is preferable to form the side sheet by hydrophobic nonwoven fabric having a liquid impermeablity or poor permeablity, a film sheet having leakage prevention property or the like. The above-mentioned sheet may be a single sheet, or two or more sheets as a combination with a functional sheet and the like.

In the absorbent body, for the constitutions and materials for the base sheet and absorbent units, it is possible to refer to Japanese Patent Application No. 2007-316239 (JP-A-2009-136498) and Japanese Patent Application No. 2007-332419 or its counterpart EP 2 226 046 A1 (prior art under Art. 54(3) EPC only).

Although an example for application to a sanitary napkin has been shown in the above-mentioned embodiment, the absorbent body is not limited to such absorbent article, and can also be applied to disposable diapers, diapers for infants or elders, and other incontinence pants and the like.

As explained above, the absorbent body of the first embodiment deforms flexibly along the shapes of various human bodies and finely fits the human body shape of a wearer, and does not provide uncomfortable feeling during wearing. Furthermore, various effects that the absorbent body finely follows the deformation of a wearing portion by motions, decrease of absorbing performance due to the deformation is not caused, and the like are provided.

(Second embodiment)

Hereinafter the second embodiment of the present invention is explained, but the explanation of the parts that overlap that of the above-mentioned first embodiment are omitted. In addition, the above-mentioned first embodiment and the present second embodiment are not in an independent and exclusive relationship, and for example, the second embodiment can be positioned as being encompassed in the more specific concept of the above-mentioned first embodiment. Furthermore, the invention according to the first embodiment and the invention according to the second embodiment are in a technical relationship in which they are linked so as to form a single general inventive concept by having the same or corresponding special technical features among them. Regarding the symbols in the drawings, an identical symbol is used for corresponding elements and the like (however, the size and detailed positional relationship follow the drawing that corresponds to each embodiment).

First, the feature that should be emphasized in the present second embodiment is that spaces Q in each of which a base sheet constitute a bottom surface in a planar view are formed between absorbent units 3, 3, 3 ... and an absorbent material 6 is disposed in the spaces Q so that the material has a lower basis weight than that of the absorbent units 3. By constituting the material of the absorbent units 3 and the absorbent material 6 that is laid on the spaces Q by such allocation of basis weights, the absorbent velocity in the thickness direction is improved, and the diffusion property of a liquid that flows in the space parts is improved. Furthermore, also in the case where the absorbent units 3 are not in liquid-current-to-pass with each other by motion or deformation of the absorbent body, the effect of diffusing the liquid is improved as compared to that having no absorbent material in the spaces. The absorbent material 6 in this case preferably has similar components to that constituting the absorbent units 3 such as pulp fibers and a superabsorbent polymer but the components of the absorbent material 6 are not necessarily limited thereto, and the absorbent material having a different composition from that of the constitutional material of the absorbent units 3 may be disposed. In the present embodiment, the absorbent material 6 is constituted by pulp fibers 6a and beads 6b of a superabsorbent polymer. For example, the basis weight of the absorbent unit 3 is adjusted to 20 to 800 $g/cm^2$, more preferably to 100 to 600 $g/cm^2$. Furthermore, the basis weight of the absorbent material 6 in the parts of the spaces Q between the absorbent units 3 is preferably adjusted to from 2 to 500 $g/cm^2$, more preferably adjusted to from 5 to 300 $g/cm^2$ in view of optimization of the above-mentioned properties. Furthermore, the ratio of the two basis weights (the percentage of the value obtained by dividing the basis weight of the absorbent material 6 in the spaces Q with the basis weight of the absorbent units 3) is preferably adjusted to 80% or less, more preferably adjusted to 50% or less, further preferably adjusted to 30% or less, and specifically preferably adjusted to 10% or less. Although the lower limit value of the above-mentioned basis weight ratio is not specifically limited, the value is practically 0.1 % or more.

In the present embodiment, since a small amount of the absorbent material 6 such as pulp and SAP is laid on the spaces Q as mentioned above, the hydrophilicity of the entirety of the absorbent body is increased. Therefore, the velocity of intaking of a liquid from the surface material is increased. Furthermore, when a small amount of the absorbent material 6 such as pulp and SAP is present in the spaces, the liquid easily spreads through the spaces. Namely, since the diffusibility of the liquid can be ensured to some extent without liquid-current-to-pass, the absorbing performance of the base is improved. Furthermore in case where spaces are in liquid-current-to-pass with each other diffusion of the liquid is further improved.

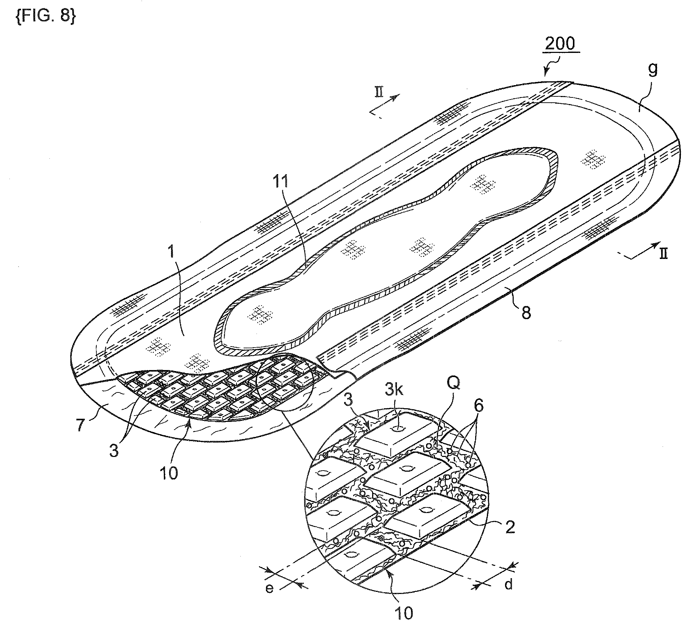

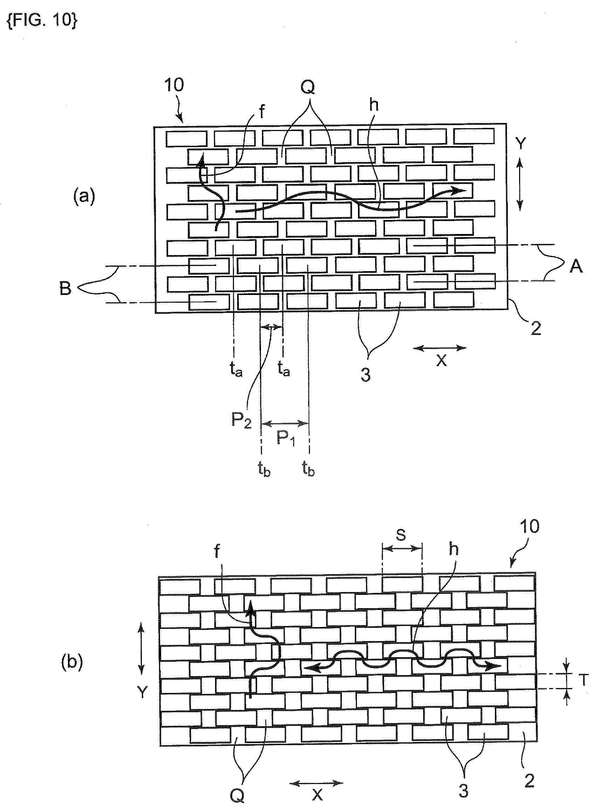

The relationship between the absorbent units 3 and fixing unit 4 is similar to that of the above-mentioned first embodiment. The dynamic function thereof is approximately similar, but is explained below in line with the present embodiment. By fixing the absorbent units 3 on the base sheet 2 only by fixing unit 4, gaps d and e between the absorbent units 3 are bent or the like, whereas the respective absorbent units 3 on the base sheet 2 are affected little by the base sheet 2 and cause little change in shape. Conversely, deformability that the base sheet 2 as the entirety of the absorbent body is bent or the like is ensured. Specifically, for example, when the sanitary napkin 200 shown in FIG. 8 is worn, the absorbent units approach to each other by bending or shrinking of the absorbent body, whereby the intervals d and e are narrowed or broaden. In this manner, the absorbent units 3, 3, 3 ... maintain their interaction for transmitting a liquid or the like while the entirety of the absorbent body 10 is deformed, whereby show fine retention of absorption of a liquid or the like. In this case, specifically in the present embodiment, the absorbent material 6 is laid between the absorbent units 3, 3, 3 .... Therefore, the hydrophilicity of the entirety of the absorbent body is improved, and the velocity of intaking of a liquid from the surface material is increased. Furthermore, due to the presence of the absorbent material 6 between the absorbent units 3, the liquid transmission property between the absorbent units 3 is realized more certainly, and thus the absorbent units 3 are not deformed when the entirety of the absorbent body 10 as mentioned above is deformed such as stretching, and a function of transmitting a liquid or the like is also provided when the absorbent units are separated from each other through the intervals c and d (spaces Q). Furthermore, since the absorbent body 10 of the present embodiment is constituted by an assembly of many small independent absorbent units, it has extremely fine "fittability to body" and "followability to motion". This point is similar to that previously mentioned in the first embodiment. Furthermore, the staggered arrangement, preferable sizes of the transverse width T, longitudinal width S, pitches $P_2$ and gaps d and e, and moreover the junction structures of the respective elements shown in FIG. 10 are also similar to those in the above-mentioned first embodiment.

Although the constitutional materials for the absorbent unit 3 are not specifically limited, those mentioned in the above-mentioned first embodiment can suitably be used. However, in the present embodiment, it is preferable to apply a sheet having stretchability as the base sheet. The preferable range, measurement method and the like of the stretchability of the base sheet 2 are similar to those mentioned in the first embodiment. The topsheet, the constitution and materials for the absorbent units, the examples of application, the advantage relating to the fitting property thereof are similar to those of the above-mentioned first embodiment.

According to the absorbent articles and the further modified examples of the above-mentioned respective embodiments, even they are folded in three or the like in individual packaging and the forms are maintained for several months in distribution and selling, the folding lines formed by folding so are hard to remain, and the articles quickly restore to their original forms, and thus the above-mentioned fine fittability to body and followability can be obtained from initiation of wearing. The function in this regard is explained below.

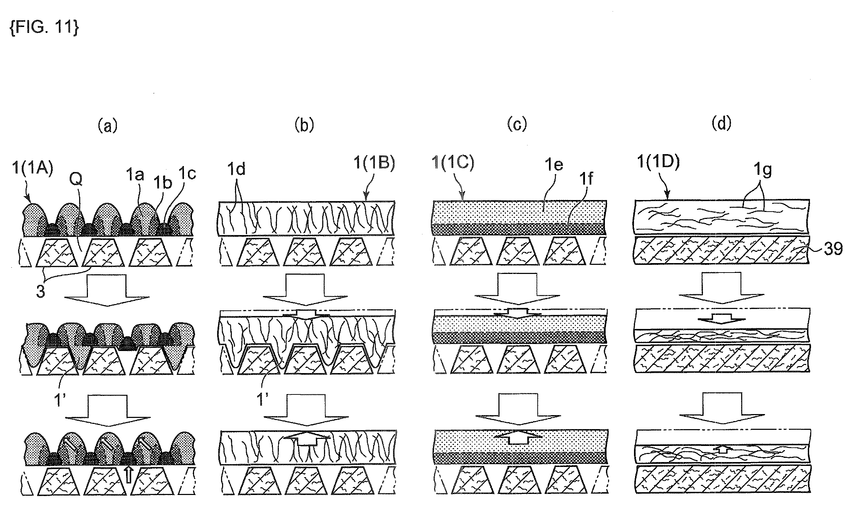

In the sanitary napkin of the present embodiment, a plurality of absorbent units (absorbent small elements) 3 are arranged with intervals, and the spaces Q are formed therebetween. Therefore, wrinkles are hard to be formed on the topsheet even the napkin is in a folded shape. Furthermore, as mentioned below, since a part of the topsheet is housed in the spaces Q during compression, the applied pressure is relaxed, and the bulk of the topsheet is readily restored when the sheet is released from compression. In addition, since the cross-sectional surface of the absorbent unit 3 is a trapezoid shape that is tapered toward the side of the topsheet as in the present embodiment, a further improved effect of relaxing folding lines on the topsheet can be obtained. With reference to the model shown in FIG. 11 (a) as an example of the present embodiment, the topsheet 1 of this example is composed of cured parts 1c, semi-cured parts 1 b and flexible parts 1a that are formed by embossing. On the other hand, the cross-sectional surface of the above-mentioned space Q is widened toward the above-mentioned topsheet in a natural shape, and when the absorbent article is individually packaged, a part of the above-mentioned topsheet, specifically the flexible parts 1 a are in a state that they are readily housed in the above-mentioned spaces Q (the housed parts of the topsheet are shown by housed portions 1' as a model). As a result, a part of the topsheet 1A deforming toward the spaces Q allows a compression force applied in the thickness direction to escape, whereby remaining of folding lines is suppressed and decrease in the volume of the topsheet is also suppressed. Furthermore, in the present embodiment, a crushed shape is also hard to be remain on the absorbent body as compared to conventional ones, and absorbability, specifically liquid passing property is kept fine, in addition to the above-mentioned significant improvement effect against folding lines on the topsheet, and thus extremely stable and high dry feeling is realized. Although the middle columns of drawings in FIG. 11 are supposed be in a folded state, these are shown by similar forms to that of the drawings on the upper and lower columns in view of comparison with that drawings.

FIG. 11 (b) is a topsheet 1 B of another example, and the constitutional fibers 1 d thereof are oriented from the surface on the top side thereof to a surface on the back side thereof.

FIG. 11 (c) is a topsheet of another example, and the topsheet has a structure for an improvement of the restoration property of the fibers themselves and for a stiffness at bonding parts of the fibers by increasing the diameters of the fibers used for the surface on the top side among the surface of the top side and the surface of the back side. Specifically, when the stiffness at the bonding portions is increased, a mesh structure that accelerates partial maintenance of the volume of the topsheet by the spaces Q or restoration of the volume of a part compressed by a low compression part (tight bonding that can restore distortion by compression) is formed in combination with the absorbent body, which lead to exhibition of a higher effect.

On the other hand, FIG. 11 (d) shows a conventional topsheet 1 D and the fibers thereof 1g, and many fibers are oriented in the planar direction of the sheet. In a conventional structure comprising a combination of such topsheet and a planar absorbent body 39 having no spaces, the topsheet that has been folded and compressed cannot restore the thickness thereof.

Although a sheet having conventionally-known materials and constitution can be used for the topsheet 1, it is preferably a nonwoven fabric made of synthetic fibers, specifically a nonwoven fabric obtained by fusion-bonding of intersection points of fibers by an air-through process or steam-jet process so as to further improve an effect of decreasing crushing against a compression force, in view of restoration of the thickness of a site that has been affected by a compression force.

Furthermore, based on FIG. 11, an example of the topsheet and the effect of restoring the thickness thereof are explained in detail.

(1) A convexo-concave structure that forms convex portions, specifically independent convex portions (FIG. 11 (a))
(2) Constitutional fibers having high thickness orientation (orientation from the surface on the top side to the surface on the back side) (FIG. 11 (b))
(3) The average liter-fiber distance is larger on the top side than on the back side (FIG. 11 (c))

In the topsheet 1A of (1) shown in FIG. 11 (a), when the flexible parts 1a that constitute the convex portions are present on the parts on which the low density absorbent material 6 is disposed, the parts are hard to fall into the spaces Q during use, and the thickness is readily maintained as a whole. Furthermore, not only that, in case where if a nonwoven fabric in which fiber intersections have been fixed by heat-fusion-bonding is used, an effect that the thicknesses of compressed parts (semi-cured parts 1 b and cured parts 1 c) are readily restored by the thickness of the flexible parts 1 a that constitute the convex portions is exhibited. In a ridge-furrow structure body in which convex portions are continued in one direction, if thickness restoration is weaken by the effect of the crushed convex portions due to continuous rib portions (convex portions), it is more preferable to apply a ridge- furrow structure body having a format of individual and independent convex portions. Furthermore, in the case when the concave portions are adjusted to have a higher density than that of the convex portions by embossing or the like, a form having independent convex portions is preferable since the semi-cured parts 1 b and cured parts 1 c in the area of the concave portions have structures that are hard to be crushed as compared to the convex portions and can be disposed so that the concave portions have parts that are overlapping with the absorbent units, whereby the height of the absorbent units at the overlapped sites becomes lower than that of the surrounding absorbent units and gaps that suppress crushing of the topsheet are readily formed.

In the topsheet 1 B of (2) shown in FIG. 11 (b), although the inclined portions of the convex portions of the convexo-concave structure may also have similar structures, the convex portions preferably have shapes that are projected in aligned parallel from embossed concave portions described in Japanese Patent Application No. 2008-331374, and a nonwoven fabric in which fibers have been oriented in the longitudinal direction by blowing gas flow by an air-through process or steam-jet process is preferable.

In the topsheet 1C of (3) shown in FIG. 11 (c), the average inter-fiber distance ($l_e$) of a running-parallel fiber group layer 1e is set to be larger than the average inter-fiber distance (If) of a crossed fiber group layer ($l_e > l_f$). As a method for providing different average inter-fiber distances between the top side and backside, a method for thickening fibers to be used, a method for selecting fiber resin materials, or the like is preferably adopted, whereby an effect of high restoration property from crushing by a mesh structure that is obtained by increasing the stiffness of the bonded portions of the fibers is possessed; however, considering the viewpoint of the texture of the nonwoven fabric, a method for improving the resin volume of the fusible parts in the fibers, as well as fibers that increase the fusion bonding areas on the bonded parts, are more preferable since the inter-fiber distance on the top side is increased by possessing parallel bonding parts on the top side, and the structure of the nonwoven fabric becomes a structure body that is formed to be readily restored from crushing.

By having one or more of the above-mentioned structures (1) to (3) of the topsheet, an absorbent article having finer absorbability, specifically liquid passing property even after unwrapping an individual packaging form can be obtained. Furthermore, as a means for improving liquid passing property in the topsheet, formation of the topsheet that is hydrophilic as a whole but hydrophobic parts and hydrophilic parts are comparted in a planar view may be exemplified, which is preferable since dry feeling of the topsheet can be improved. Furthermore, it is further preferable to dispose the convex portions of the convexo-concave structure on the hydrophobic side and the concave portions on the hydrophilic side, since transfer of a liquid in the topsheet can further be improved and dry feeling can further be improved.

In the present embodiment, the density ($D_1$) of the topsheet 1 is set to be lower than the density ($D_3$) of the absorbent units 3. In this manner, due to a high capillary force arising from to the high density ($D_3$) of the absorbent units 3 ($D_3 > D_1$), the liquid passing property of the topsheet 1 can be improved, and the remaining amount of liquid can also be decreased. Furthermore, in the present embodiment, since the parts on which the absorbent material 6 that is constituted by similar materials to those of the absorbent units 3 and has a low density is disposed have higher surface hydrophilicity than that of the fiber material in the topsheet 1, they can pull in the liquid from the contact surface of the fiber. As a result, the whole surface of the absorbent body readily pull in the liquid from the topsheet, whereby the amount of the remaining liquid on the topsheet can be decreased and dry feeling can further be improved. Furthermore, the case when the density of the topsheet 1 is higher than the density of the absorbent material (low density part) 6 ($D_3 > D_1 > D_6$) is more advantageous due to difficulty in crushing of the topsheet.

Unless otherwise specified, the densities mean values measured as follows.

(1) Density of absorbent unit ($D_3$)

Since a density is obtained by dividing a weight (g) by a volume ($cm^3$), the weight and volume of the absorbent unit are measured. The weight is measured after cutting into a size of 5 cmx5 cm. (The value obtained from an arithmetic average value of values obtained from three parts, or from three absorbent articles is defined as the weight of the sample.) For the volume, the sample whose weight has been measured is used, and a high accuracy shape measurement system KS-1100 (trade name) and KS-Analyzer (trade name) manufactured by Keyence Corporation are used. The settings of KS-1100 during the measurement were as follows: measurement initiation position (X=0, Y=0), measurement range (X=40,000 $\mu$m, Y=40,000 $\mu$m), measurement pitch (X=20 $\mu$m, Y=20 $\mu$m) and transfer velocity: 7,500 $\mu$m. The KS-Analyzer is used after correcting measured data by "correction" menu. The setting of the correction was performed at a size of 3x3, a running number of times of 1 and a degree of 100. Then, the measurement is performed on the whole area of the "measurement" menu (thus, the measurement range is 4 cm$\times$4 cm), and a density is obtained from the weight and volume by using the average value of the three samples that has been cut out as a volume. In the case when the shape of the cross-sectional surface is such as a trapezoid and semicircle which can be grasped from the top surface, a table on which the sample is to be set is defined as a base line (0 value), whereas in the case when the cross-sectional surface cannot be measured from only the top surface such as a hexagon (FIG. 12 (c)), a suitable volume value is obtained by suitably setting the base line when the measurement is performed from the upper and bottom surfaces, or by using the total value obtained by measuring individual absorbent units.

In addition, the outline parts of the absorbent units sometimes get loose during the production, and in such case, the parts have slightly infinite forms, or segmentation from the absorbent material becomes ambiguous. In such case, the measurement is performed by the treatment described in (3) Fiber density of absorbent material ($D_6$) mentioned below.

(2) Fiber density of topsheet ($D_1$)

In the case of a planar topsheet, the density can be measured from the weight and volume by using the weight, measurement size and $T_0$ obtained by the measurement using KES-G5 Handy Compression Tester, whereas in case where the topsheet has a convexo-concave structure, the density ($D_1$) of the topsheet is calculated by measuring the weight and volume in a manner similar to the above-mentioned (1).

In addition, in the case where an intermediate unit such as an intermediate sheet is present between the topsheet and absorbent unit, the average density of the members on the side of the topsheet including the intermediate unit is defined as the above-mentioned density ($D_1$).

(3) Density of absorbent material ($D_6$)

The density $D_6$ of the absorbent material is calculated before the measurement of the above-mentioned absorbent unit. A measurement sample size of 5 cm $\times$ 5 cm is cut out and a weight thereof is measured, and a volume thereof is measured by using a high accuracy shape measurement system KS-1100 and KS-Analyzer manufactured by Keyence Corporation; thereafter the weight and volume of the sample from which the above-mentioned absorbent material between the absorbent units has been removed are measured, and the weight and volume of the absorbent units are subtracted from the original weight and volume, whereby the weight and volume of the absorbent material can be obtained and the density $D_6$ can be calculated. The absorbent material between the absorbent units is preferably removed by using tweezers or the like. This is to prevent disarrangement of the surface of the absorbent units, which caused to disable differentiation between the absorbent units and from the absorbent material, by an outer force such as wind force, and since the fibers and the like are conveyed by air, deposited and formed to the absorbent units, peeling of the surfaces of the absorbent units due to removal by using tweezers generally may not occur.

The outline parts of the absorbent units sometimes get loose during the production, and in such case, the parts have slightly infinite forms, or segmentation from the absorbent material becomes ambiguous. In such case, removal by tweezers is tried after the measurements of the weight and volume, and in the case where only the loosen parts can be removed, they are handled as the absorbent material, and the weight and volume of the absorbent units are measured again.

In either of the above-mentioned calculations of the density of the members, parts in which the constitutional material is not present such as open pores are not included for measurement, whereas predetermined areas comprising relatively minute structural parts in the convex portions and concave portions are demarcated and calculated, and the density is calculated as the density of each of structural portions averaging the convex portions and concave portions as an average density.

According to the present embodiment, since a density gradient between the topsheet and the absorbent units is set as mentioned above, in the case where a liquid that has passed through the topsheet is little, the individual absorbent units absorb the liquid quickly. On the other hand, in the case where the liquid that has passed through the topsheet is much, since the spaces Q or the absorbent material 6 laid thereon are present on the parts on which the individual absorbent units are separated from each other, the liquid can be temporarily stocked on these parts. Therefore, the liquid is hard to diffuse on the topsheet even when excretion of menstrual blood or the like is much, whereby dry feeling on the topsheet can be improved.

As mentioned above, generally in an individual package in which the absorbent article is folded or a package form in which individual absorbent articles are superposed, the state in which a pressure is applied in the thickness direction of the absorbent article continues. Therefore, the thicknesses of the topsheet and the absorbent body are crushed, and the space in which a liquid passes easily is crushed due to specifically decrease in bulkiness in the topsheet. Then, the passage resistance of the liquid increases, and the residual liquid on the topsheet significantly increases. On the other hand, according to the present embodiment, even in the case of the individual packaging or the package form, a pressure is hard to be applied to the topsheet since spaces that can house a part of the topsheet are formed on the absorbent body, whereby stable wearing performance in which the passage property of the liquid is hardly spoiled can be realized.

FIG. 12 is an enlarged cross-sectional view that schematically shows the states of the absorbent units and absorbent material according to the respective embodiments of the absorbent article of the present invention. Absorbent bodies having various forms as shown in the figure can be used. In the embodiment (a) of the absorbent units 3 each having a trapezoid cross-sectional surface as in the above-mentioned first embodiment, dimples that correspond to the individual absorbent units 3 are formed in a fiber stacking drum during the production. When the absorbent units are transferred to the backsheet, it is preferable to push the absorbent units out by air since the absorbent units 3 can be formed accurately on the above-mentioned spaces Q. Furthermore, by pushing the air out, the peripheral parts of the absorbent units 3, loose and whereby trapezoidal parts having a high density, which remain thereby and parts on which the absorbent material 6 having a low density is disposed, are formed. Since the absorbent material 6 often sinks toward the side of the backsheet and has dimples in the case where the amount of the air that is pushed off is small, it is preferable to increase the amount of the air that is pushed off so as to make the entirety of the absorbent body approximately planar. Alternatively, in view of forming more surely, it is preferable to blow air flow from the side of the topsheet or the side of the backsheet in a step that is different from the step of the fiber stacking drum. In this case, it is preferable to perform after transferring and fixing the absorbent units to the base sheet 2 in view of eliminating disorder of the arrayed absorbent units 3 and improving passage of the air.

FIG. 12 (b) and (c) show the cross-sectional surface shapes of the absorbent units 3 that can be formed by a similar method. Since in the embodiment shown in (b) (the absorbent units) have semicircular cross-sectional surface shapes, it is in a low-contact state with the topsheet, whereby crushing of the topsheet by compression can be relatively decreased as compared to trapezoids. In the embodiment shown in (c), gaps or low density parts for diffusing a liquid are disposed also on the side of the backsheet, whereby a structure that is hard to be affected by a wearing state (mainly by a wearing pressure) can be provided.

FIG. 13 is an enlarged perspective view that schematically shows the arrangements of the absorbent units according to the respective embodiments of the absorbent articles of the present invention. In (a) to (c) in the figure, the arrangements of the absorbent units of the absorbent bodies are shown as states having no absorbent material 6. In the forms in which the spaces Q do not have the absorbent material 6 as just described, the above-mentioned pushing out by air flow is more decreased during the production thereof to decrease loosing of the surfaces of the absorbent units 3 and transfer-adhesion is performed by an adhesive that has been applied to the backsheet 7, and thus diffusion of a liquid by the gaps of the absorbent body become more predominant. This leads to improvement of dry feeling of the topsheet of the absorbent article, and the residual amount of the liquid is also decreased since the topsheet 1 follows the individual shapes on the side of the topsheet of the absorbent units 3.

FIG. 14 is a cross-sectional view that schematically shows the sanitary napkin according to a further different embodiment of the absorbent article of the present invention. The figure shows, as the fourth embodiment, the cross-sectional view of a sanitary napkin 300 comprising a topsheet and an absorbent body, and an intermediate sheet 9 disposed therebetween. This sanitary napkin 200 has a similar structure to that of the sanitary napkin 200 of the second embodiment except that the intermediate layer is disposed, and the different part is explained below.

As the intermediate sheet 9, a nonwoven fabric obtained by an air-through process or steam-jet process as in the case of topsheet, as well as a sheet on which open pores (slits) have been formed and the like can be used. In view of transitivity of the liquid (decrease in the amount of the residual liquid), the intermediate sheet preferably has no convexes and concaves, and the fiber density ($D_9$) thereof is preferably higher than the fiber density ($D_1$) of the topsheet and is preferably lower than the fiber density ($D_3$) of the absorbent units 3 ($D_3>D_9>D_1$). In addition, the fiber density of the intermediate sheet can be measured by a similar manner to that of the above-mentioned topsheet.

Since the intermediate sheet 9 is disposed, the absorbent article has improved dry feeling and fine cushion feeling, and the transportation (conveyancing) property of the absorbent body during the production is stabilized. Furthermore, crushing becomes harder to be maintained against also crushing by compression. In case where a web layer that has not been formed into a nonwoven fabric by such as fusion bonding of fibers or the like, or a sheet having raised fiber fluffs is used on one side of the intermediate layer instead of the planar sheet material shown in the drawing, contacting property with the absorbent units 3 is improved even in the case when the absorbent material 6 between the absorbent units 3 is absent, or a constitution that has not been planarized by the absorbent material 6 is used, whereby the amount of the residual liquid is decreased easily.

The present invention is not to be construed as being limited to the above-mentioned respective embodiments, and for example, a plurality of the absorbent units 3 may have several kinds of thicknesses instead of the same thickness. Furthermore, a structure in which the side parts of the absorbent units are lower than the center can be adopted for the purpose of improving wearing comfort wearing, and a structure using a combination or mixture of two to three kinds of absorbent units having different heights can be adopted for the purpose of decreasing crushing of the topsheet by compression.

EXAMPLES

Hereinafter, the present invention will be described more in detail with reference to Examples, but the present invention is not limited thereto.

(Example and Comparative Example corresponding to first embodiment)

An absorbent body test body having the form shown in FIG. 7 (a) was prepared (however, the sizes of the respective units are described based on the symbols shown in FIG. 3). A pulp/superabsorption polymer mixed body ($200/50$ g/m$^2$) was used as the absorbent units, and a styrene-ethylene-propylene copolymer (SEPS, 13 g/m$^2$) nonwoven fabric was used as the base sheet. The size of each absorbent unit was transverse width T = 6.0 mm and longitudinal width S = 13.5 mm, and the absorbent units were disposed by pitches of $P_2$ = 7.5 mm, gaps of d = 1.5 mm and e = 1.0 mm. The absorbent units were fixed on the base sheet by using ultrasonic embossing (radius: 1.0 mm).

FIG. 7 (b) is a plane view of the state in which the above-mentioned absorbent body test body is extended by 30% in the length direction. In the state of the natural length, the rectangular absorbent units 3 were disposed by a staggered arrangement on the base sheet 2 while keeping approximately similar gaps in the longitudinal direction and transverse direction. When the entirety of the absorbent body 10 was extended in the longitudinal direction (direction X) of the absorbent body from this state, the absorbent body 10 was shrunk in the transverse direction (direction Y). In this test example, the extension rate in the longitudinal direction was 30%, whereas the shrinkage rate in the transverse width direction was 14%.

It is understood that since each of the absorbent units 3 is fixed on the base sheet 2 by only the central fixing unit 4 (FIG. 2) even in such stretching state, the individual shape of the absorbent unit is not affected and only the base sheet 2 is extended and the gaps between the absorbent units 3 in the longitudinal direction are increased. At that time, it was confirmed that the liquid was effectively transferred between the adjacent absorbent units 3 in the longitudinal and transverse directions between the adsorbent units as shown in the arrows f and h in the extending state (FIG. 3 (b)).

Next, on a stretchable base sheet having a large surface area so that the above-mentioned test body has similar shape and surface area to those of an absorbent body in a commercial general sanitary napkin, an absorbent body test body in which the number of the absorbent units were increased and the absorbent units were arranged by a staggered arrangement without changing the sizes and gaps of the individual absorbent units was prepared. A test body 101 (Example) of a sanitary napkin was prepared by incorporating the absorbent body test body prepared as mentioned above with similar topsheet, backsheet, side sheet and the like of the above-mentioned commercial sanitary napkin. For comparison, as the above-mentioned commercial sanitary napkin, a continuous body (75 mm×200 mm) of a conventional product (test body c11) (pulp/SAP ($200/50$ g/m$^2$)) was used as an absorbent body (Comparative Example). Furthermore, a general underwear fabric (cotton 45%, rayon 45%, polyurethane 10%) that was cut out in a similar shape to that of above-mentioned commercial sanitary napkin was prepared (test body s11) (Reference Example). Using these test bodies of Example, Comparative Example and Reference Example, bending moments and shear stresses were measured according to the following procedures, and the results thereof were summarized in Table 1.

(Method for measurement of bending moment)

KES-FBS-L (trade name) by Kato Tech Co., Ltd. was used as a measurement device. The measurement was performed at the maximum curvature of $\pm 0.5$ cm$^{-1}$ and a curvature deformation velocity of 0.5 cm$^{-1}$/sec (constant), and the cramp width was 40 mm and the sample width was 25 mm. The measurement was performed under conditions of a temperature of 20°C and a humidity of 65%.

(Method for measuring shear force)

KES-FB1-AUTO-A (trade name) by Kato Tech Co., Ltd. was used as a measurement device. The measurement was performed at the maximum shear amount of $\pm \tan 8°$ and a shear velocity of 0.5°/sec, and the cramp width was 5 mm and the sample width was 20 mm. The measurement was performed under conditions of a temperature of 20°C and a humidity of 65%.

Table 1

|  | Example | Reference example | Comparative example |
|---|---|---|---|
| Test Body | 101 | s11 | c11 |
| Bending moment (gf·cm/cm)[*1] | 5.8 | 4.0 | 29.5 |
| Shearing stress (gf/cm$^2$)[*2] | 14.1 | 8.0 | 60.2 |
| *1: Curvature = 0.5(cm$^{-1}$)<br>*2: Shear angle = 8.0(deg) | | | |

It is understood that one using the absorbent body according to the present invention (Example) shows a value of a bending moment that is close to the value of the underwear as compared to the conventional product (Comparative Example), and flexibly fits to various human body shapes to ensure extremely fine fittability to human body. Furthermore, one using the absorbent body according to the present invention (Example) generated only a shear stress that was close to the shear stress of the underwear, as compared to the conventional product (Comparative Example). Therefore, it is understood that the absorbent body of the present invention follows and deforms finely in accordance with the motion of a human body, exhibits extremely high followability, and does not provide uncomfortable feeling even in cases when various motions are done.

(Example and Comparative Example corresponding to second embodiment)

The absorbent body test body shown in FIG. 10 (a) was prepared (the absorbent material 6 is omitted in FIG. 3). A pulp/superabsorbent polymer mixture formed into truncated pyramid (200/50 g/m$^2$) as illustrated was used as the absorbent units, and a nonwoven fabric having a transverse width of 200 mm and a longitudinal width of 100 mm was used as the base sheet. The size of each absorbent unit was transverse width T = 6.5 mm and a longitudinal width S = 13 mm, and the absorbent units were disposed by pitches of $P_2$ = 7.5 mm, gaps of d = 1.5 mm and e = 1.0 mm (see FIG. 8 and FIG. 10 for the symbols of the respective sizes). The absorbent units were fixed on the base sheet by using ultrasonic embossing (radius: 1.0 mm). An absorbent material composed of a similar material to that of the absorbent units, which had not been formed, was transported by air to the spaces extending between the absorbent units and laid between the absorbent units. The ratio of the basis weight of the absorbent units and the basis weight of the absorbent material (basis weight ratio) can be obtained by the following procedures.

1. The basis weight of the entirety of the absorbent body (the absorbent material on the absorbent units (truncated pyramid) + the nonwoven fabric + absorbent materials in spaces between the absorbent units) is measured.
2. Ten absorbent units each having a truncated pyramid shape are arbitrarily cut out in the state of including the nonwoven fabric, and the basis weights thereof are measured and an average value is obtained. Thereafter the basis weight of the nonwoven fabric that has been measured in advance subtracted therefrom leaves the basis weight of the absorbent unit.
3. The basis weight of the absorbent material on the spaces between the absorbent units is calculated by: the basis weight of the absorbent material on the spaces between the absorbent units=the entire basis weight-the basis weight of the nonwoven fabric-the basis weight of the absorbent units.
4. The ratio of the basis weight of the absorbent units to the basis weight of the absorbent material (basis weight ratio) is calculated by:

{(the basis weight of the absorbent material on the spaces between the absorbent units)/(the basis weight of the absorbent unit)}×100 (%).

In the above-mentioned absorbent body test body, a test body using SMS nonwoven fabric (manufactured by Unitika Ltd., trade name; Eleves) as a material for constituting the base sheet (test body 201: Example) and a test body using stretchable MB nonwoven fabric (resin: manufactured by Kuraray Corporation, trade name; Septon, obtained by forming Septon into a nonwoven fabric by using a meltblown apparatus of the company) (test body 202: Example) were prepared respectively. For comparison with these, an absorbent body utilized in commercial sanitary napkins, which was cut into a similar shape to that of the above-mentioned test bodies (Comparative Example: test body c21) was prepared. Using these test bodies of the Examples and Comparative Example, flexural rigidity were measured in accordance with the following procedures, and the results were summarized in Table 2.

(Method for measurement of flexural rigidity)

KES-FBS-L (trade name) by Kato Tech Co., Ltd. was used as a measurement device. The measurement was performed at the maximum curvature of $\pm 0.5$ cm$^{-1}$ and a curvature deformation velocity of 0.5 cm$^{-1}$/sec (constant), and the cramp width was 40 mm and the sample width was 25 mm. The measurement was performed under conditions of a temperature of 20°C and a humidity of 65%.

Table 2

|  | Example | Example | Comparative example |
|---|---|---|---|
| Test body | 201 | 202 | c21 |
| Absorbent unit | pulp/SAP | ← | ← |
| Size of absorbent unit | 6.5 mm $\times$ 13mm | ← | - |
| Ratio of absorbent units [*1] | 80% | ← | - |
| Basis weight ratio [*2] | 8% | ← | - |
| base sheet | SMS nonwoven fabric | Stretchable MB nonwoven fabric | - |
| Flexural rigidity MD | 10 gf·cm/cm | 8 qf·cm/cm | 30 gf·cm/cm |
| (curvature 0.5 cm$^{-1}$) CD | 8 gf·cm/cm | 5 gf·cm/cm | 17 gf·cm/cm |

*1 Ratio of absorbent units: the percentage of the value obtained by dividing the occupied surface area of the absorbent units in a planar view by the surface area of the entirety of the absorbent body.
*2 Basis weight ratio: the percentage of the value obtained by dividing the basis weight of the absorbent material between the absorbent units (spaces) by the basis weight of the absorbent unit.

In the case of the absorbent body test body c21 (Comparative Example) in the conventional product, high flexural rigidity was shown in both of the MD direction and CD direction. On the other hand, in the absorbent bodies according to the present invention (test bodies 201 and 202, Examples), the flexural rigidity was significantly decreased in both of the MD direction and CD direction. Therefore, it is understood that, according to the absorbent body of the present invention, the absorbent body finely follows and deforms in accordance with the motion of a human body, exerts extremely high followability, and does not give uncomfortable feeling in the case when various motions are performed.
FIG. 10 (b) is a schematic plane view that shows the state when the above-mentioned absorbent body test body 202 is extended in the longitudinal direction. In this test example, the extention rate in the longitudinal direction was 30%, whereas the shrink rate in the transverse width direction was 14%. It was confirmed that the liquid between the adjacent absorbent units 3 was effectively transferred in the longitudinal and transverse directions as shown by the arrows f and h, in both of the non-extending state (FIG. 10 (a)) and extending state (FIG. 10 (b)).

DESCRIPTION OF SYMBOLS

1          Topsheet

| 2 | Base sheet |
|---|---|
| 3 | Absorbent unit |
| 4 | Fixing unit |
| 6 | Absorbent material |
| 6a | Pulp fiber |
| 6b | Beads of superabsorbent polymer |
| 7 | Backsheet |
| 8 | Side sheet |
| 9 | Intermediate sheet |
| 10 | Absorbent body |
| 11 | Groove |
| 100, 200, 300 | Absorbent article |

**Claims**

1. An absorbent body, comprising:

    a base sheet (2); and
    a plurality of absorbent units (3) separated from each other and each having attached to the base sheet (2) via a fixing unit (4),
    wherein each of the fixing units (4) is positioned in the area of each of the absorbent units (3) in a planar view, and the absorbent units (3) are arranged in a staggered manner in which rows of a plurality of absorbent units (3) are disposed in parallel along a longitudinal direction (X), and the rows that are adjacent with respect to a transverse direction (Y) are shifted with each other, so that, when an elongation force is applied in the longitudinal direction (X) to the absorbent body, the absorbent units (3) approach or contact with each other, thereby allowing liquid current to pass with each other,
    wherein the plurality of absorbent units (3) on each row is disposed with a pitch ($P_1$), and the rows that are adjacent with respect to the transverse direction (Y) are shifted by a half of said pitch ($P_1$) with each other.

2. An absorbent body, comprising:

    a base sheet (2); and
    a plurality of absorbent units (3) separated from each other and each having attached to the base sheet (2) via a fixing unit (4),
    wherein each of the fixing units (4) is positioned in the area of each of the absorbent units (3) in a planar view, and an absorbent material (6) is disposed in spaces (Q) between the absorbent units (3) so that they allow liquid current to pass with each other, the absorbent material (6) having a basis weight that is smaller than the basis weight of the absorbent units (3).

3. The absorbent body according to Claim 1 or 2, wherein the absorbent unit (3) has a top surface and a bottom surface that is contacted with the base sheet (2), and the surfaces have different surface areas in a planar view.

4. The absorbent body according to any one of Claim 1 to 3, wherein the absorbent units (3) are disposed on a surface of the base sheet (2) in longitudinal and transverse directions (X, Y) in such a manner that they have predetermined gaps d and e with each other in a natural state, and the gaps d and e are from 0.1 to 30 mm, and from 0.1 to 30 mm, respectively, and the base sheet (2) is a stretchable sheet.

5. The absorbent body according to any one of Claims 1 to 4, wherein the absorbent unit (3) has a rectangular shape in a planar view.

6. The absorbent body according to any one of Claims 1 to 5, wherein the absorbent unit (3) has a constricted polygonal form in a planar view.

7. The absorbent body according to any one of Claims 1 to 6, wherein a space in which the base sheet (2) constitutes a bottom surface is formed between the absorbent units (3) in a planar view.

**8.** The absorbent body according to any one of Claims 1 to 7, wherein the absorbent units (3) are arranged on one surface side of the base sheet (2).

**9.** The absorbent body according to Claim 2, wherein the absorbent material (6) is the same as a material that constitutes the absorbent units (3).

**10.** The absorbent body according to Claim 2 or 9, wherein a space in which the base sheet constitutes a bottom surface is formed between the absorbent units (3) in a planar view, and the absorbent material (6) is disposed on the space.

**11.** The absorbent body according to Claim 2, wherein rows of a plurality of absorbent units (3) are disposed in parallel along a longitudinal direction (X), the plurality of absorbent units (3) on each row being disposed with a pitch (P), and the rows that are adjacent with respect to a transverse direction (Y) are shifted by a half of said pitch ($P_1$) with each other.

**12.** An absorbent article comprising the absorbent body according to any one of Claims 1 to 11.

**Patentansprüche**

**1.** Saugfähiger Körper mit:

einer Basisschicht (2); und
mehreren voneinander getrennten saugfähigen Einheiten (3), die jeweils über eine Befestigungseinheit (4) an der Basisschicht (2) angebracht sind,
wobei in einer Draufsicht in dem Bereich jeder der saugfähigen Einheiten (3) je eine der Befestigungseinheiten (4) angeordnet ist, und
die saugfähigen Einheiten (3) in einer gestaffelten Weise angeordnet sind, derart, dass die mehreren saugfähigen Einheiten (3) in sich parallel in Längsrichtung (X) erstreckenden Reihen angeordnet sind, und die saugfähigen Einheiten (3) einer Reihe mit einem Versatz zu den saugfähigen Einheiten (3) einer in Querrichtung (Y) benachbarten Reihe angeordnet sind,
so dass, wenn auf den saugfähigen Körper eine Dehnungskraft in die Längsrichtung (X) ausgeübt wird, die saugfähigen Einheiten (3) sich einander nähern oder miteinander in Kontakt kommen, um einen Flüssigkeitstransfer zu erlauben,
wobei die mehreren saugfähigen Einheiten (3) jeder Reihe in einem Intervall ($P_1$) angeordnet sind und die Reihen, die in Querrichtung (Y) benachbart sind, mit einem Versatz, der halb so groß wie das Intervall ($P_1$) ist, angeordnet sind.

**2.** Saugfähiger Körper mit:

einer Basisschicht (2); und
mehreren voneinander getrennten saugfähigen Einheiten (3), die jeweils über eine Befestigungseinheit (4) an der Basisschicht (2) angebracht sind,
wobei in Draufsicht in dem Bereich jeder der saugfähigen Einheiten (3) je eine der Befestigungseinheiten (4) angeordnet ist, und
in Zwischenräumen (Q) zwischen den saugfähigen Einheiten (3) ein saugfähiges Material (6) angeordnet ist, um dadurch einen Flüssigkeitstransfer zu erlauben, wobei das saugfähige Material (6) ein Basisgewicht hat, das kleiner ist als das Basisgewicht der saugfähigen Einheiten (3).

**3.** Saugfähiger Körper nach Anspruch 1 oder 2, wobei die saugfähige Einheit (3) eine obere Oberfläche und eine die Basisschicht (2) kontaktierende untere Oberfläche hat und in Draufsicht die Oberflächen unterschiedliche Flächengröße haben.

**4.** Saugfähiger Körper nach einem der Ansprüche 1 bis 3, wobei die saugfähigen Einheiten (3) in Längs- und Querrichtung (X,Y) an einer Oberfläche der Basisschicht (2) angeordnet sind, derart dass sie in einem natürlichen Zustand vorgegebene Abstände d und e einer Größe zwischen 0,1mm und 30mm bzw. 0,1mm und 30mm haben, und die Basisschicht (2) eine dehnbare Schicht ist.

**5.** Saugfähiger Körper nach einem der Ansprüche 1 bis 4, wobei in einer Draufsicht die saugfähige Einheit (3) rechteckförmig ist.

6. Saugfähiger Körper nach einem der Ansprüche 1 bis 5, wobei in einer Draufsicht die saugfähige Einheit (3) die Form eines eingeschnürten Polygons hat.

7. Saugfähiger Körper nach einem der Ansprüche 1 bis 6, wobei in einer Draufsicht ein Raum, dessen Bodenfläche von der Basisschicht (2) gebildet wird, zwischen den saugfähigen Einheiten (3) gebildet ist.

8. Saugfähiger Körper nach einem der Ansprüche 1 bis 7, wobei die saugfähigen Einheiten (3) an einer Oberflächenseite der Basisschicht (2) angeordnet sind.

9. Saugfähiger Körper nach Anspruch 2, wobei das saugfähige Material (6) und ein Material, aus denen die absorbierenden Einheiten (3) hergestellt sind, vom gleichen Typ sind.

10. Saugfähiger Körper nach Anspruch 2 oder 9, wobei in einer Draufsicht ein Raum, dessen Bodenfläche von der Basisschicht gebildet wird, zwischen den saugfähigen Einheiten (3) gebildet ist und das saugfähige Material (6) in dem Raum angeordnet ist.

11. Saugfähiger Körper nach Anspruch 2, wobei
die mehreren saugfähigen Einheiten (3) in sich parallel in Längsrichtung (X) erstreckenden Reihen angeordnet sind und die mehreren saugfähigen Einheiten (3) jeder Reihe in einem Intervall ($P_1$) angeordnet sind,
die Reihen, die in Querrichtung (Y) benachbart sind, mit einem Versatz, der halb so groß wie das Intervall ($P_1$) ist, angeordnet sind.

12. Saugfähiger Artikel, der einen saugfähigen Körper nach einem der Ansprüche 1 bis 11 aufweist.

## Revendications

1. Corps absorbant comprenant :

    une feuille de base (2) ; et
    une pluralité d'unités absorbantes (3) séparées les unes des autres et étant chacune fixées à la feuille de base (2) via une unité de fixation (4),
    dans lequel chacune des unités de fixation (4) est positionnée dans la région de chacune des unités absorbantes (3) sur une vue en plan, et
    les unités absorbantes (3) sont agencées d'une manière en quinconce, dans laquelle des rangées d'une pluralité d'unités absorbantes (3) sont disposées en parallèle le long d'une direction longitudinale (X), et les rangées qui sont agencées par rapport à une direction transversale (Y) sont déplacées les unes par rapport aux autres, de sorte que lorsqu'une force d'allongement est appliquée dans la direction longitudinale (X) sur le corps absorbant, les unités absorbantes (3) se rapprochent ou sont en contact entre elles, permettant ainsi au flux de liquide de passer des unes aux autres,
    dans lequel la pluralité d'unités absorbantes (3) sur chaque rangée sont disposées avec un écartement ($P_1$), et les rangées qui sont adjacentes par rapport à la direction transversale (Y) sont déplacées d'une moitié dudit écartement ($P_1$) les unes par rapport aux autres.

2. Corps absorbant comprenant :

    une feuille de base (2) ; et
    une pluralité d'unités absorbantes (3) séparées les unes des autres et étant chacune fixées à la feuille de base (2) via une unité de fixation (4),
    dans lequel chacune des unités de fixation (4) est positionnée dans la région de chacune des unités absorbantes (3) sur une vue en plan, et
    un matériau absorbant (6) est disposé dans des espaces (Q) entre les unités absorbantes (3) de sorte qu'ils permettent au flux de liquide de passer des unes aux autres, le matériau absorbant (6) ayant un poids de base qui est inférieur au poids de base des unités absorbantes (3).

3. Corps absorbant selon la revendication 1 ou 2, dans lequel l'unité absorbante (3) a une surface supérieure et une surface inférieure qui est en contact avec la feuille de base (2), et les surfaces ont des régions de surface différentes sur une vue en plan.

4. Corps absorbant selon l'une quelconque des revendications 1 à 3, dans, lequel les unités absorbantes (3) sont disposées sur une surface de la feuille de base (2) dans les directions longitudinale et transversale (X, Y) de sorte qu'elles ont des interstices prédéterminés d et e entre elles à l'état naturel, et les interstices d et e mesurent de 0,1 à 30 mm et de 0,1 à 30 mm respectivement, et la feuille de base (2) est une feuille extensible.

5. Corps absorbant selon l'une quelconque des revendications 1 à 4, dans lequel l'unité absorbante (3) a une forme rectangulaire sur une vue en plan.

6. Corps absorbant selon l'une quelconque des revendications 1 à 5, dans lequel l'unité absorbante (3) a une forme polygonale étroite sur une vue en plan.

7. Corps absorbant selon l'une quelconque des revendications 1 à 6, dans lequel un espace dans lequel la feuille de base (2) constitue une surface inférieure, est formé entre les unités absorbantes (3) sur une vue en plan.

8. Corps absorbant selon l'une quelconque des revendications 1 à 7, dans lequel les unités absorbantes (3) sont agencées sur un côté de surface de la feuille de base (2).

9. Corps absorbant selon la revendication 2, dans lequel le matériau absorbant (6) est le même qu'un matériau qui constitue les unités absorbantes (3).

10. Corps absorbant selon la revendication 2 ou 9, dans lequel une espace dans lequel la feuille de base constitue une surface inférieure, est formé entre les unités absorbantes (3) sur une vue en plan, et le matériau absorbant (6) est disposé sur l'espace.

11. Corps absorbant selon la revendication 2, dans lequel les rangées d'une pluralité d'unités absorbantes (3) sont disposées en parallèle le long d'une direction longitudinale (X), la pluralité d'unités absorbantes (3) sur chaque rangée étant disposées avec un écartement ($P_1$), et
les rangées qui sont adjacentes par rapport à une direction transversale (Y) sont déplacées d'une moitié dudit écartement ($P_1$) les unes par rapport aux autres.

12. Article absorbant comprenant le corps absorbant selon l'une quelconque des revendications 1 à 11.

{FIG. 1}

EP 2 433 602 B1

{FIG. 2}

{FIG. 3}

(a)                                    (b)

{FIG. 4}

(a)

(b)

{FIG. 5}

(a)

(b)

{FIG. 6}

{FIG. 7}

(a)　　　　　　　　　　　　　　　　　　　　　(b)

{FIG. 8}

EP 2 433 602 B1

{FIG. 9}

{FIG. 10}

(a)

(b)

{FIG. 11}

(a)　　　　　　　　(b)　　　　　　　　(c)　　　　　　　　(d)

{FIG. 12}

(a)

(b)

(c)

{FIG. 13}

(a)

(b)

(c)

EP 2 433 602 B1

{FIG. 14}

## REFERENCES CITED IN THE DESCRIPTION

**Patent documents cited in the description**

- JP 3558801 B
- JP 2703596 B
- JP 4173656 B
- JP 2003103677 A
- EP 2014269 A1
- EP 0947549 A1
- JP 2007316239 A
- JP 2009136498 A
- JP 2007332419 A
- EP 2226046 A1
- JP 2008331374 A